(19) Europäisches Patentamt
European Patent Office
Office européen des brevets

(11) Veröffentlichungsnummer : **0 153 270**
**B1**

(12) # EUROPÄISCHE PATENTSCHRIFT

(45) Veröffentlichungstag der Patentschrift : **27.07.88**

(51) Int. Cl.⁴ : **C 07 J 53/00**, A 61 K 31/57

(21) Anmeldenummer : **85730005.7**

(22) Anmeldetag : **17.01.85**

(54) **1alpha, 2alpha-Methylen-6-methylen- und 6alpha-methyl-pregnene, Verfahren zu deren Herstellung und diese enthaltende pharmazeutische Präparate.**

(30) Priorität : **20.01.84 DE 3402330**

(43) Veröffentlichungstag der Anmeldung : **28.08.85 Patentblatt 85/35**

(45) Bekanntmachung des Hinweises auf die Patenterteilung : **27.07.88 Patentblatt 88/30**

(84) Benannte Vertragsstaaten : **AT BE CH DE FR GB IT LI LU NL SE**

(56) Entgegenhaltungen :
**DE-A- 1 097 986**
**DE-A- 1 101 415**
**DE-A- 2 047 071**
**FR-A- 1 404 683**
**US-A- 3 112 305**
**ARZNEIMITTEL FORSCHUNG, Band 17, Nr. 9, September 1967, Seiten 1103-1116, Aulendorf, DE; R. Wiechert et al.: "Wirkungen und Struktur neuer antiandrogener Steroide"**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Annen, Klaus, Dr.**
**Osthofstrasse 80**
**D-4400 Münster-Albachten (DE)**
Erfinder : **Laurent, Henry, Dr.**
**Glambecker Weg 21**
**D-1000 Berlin 28 (DE)**
Erfinder : **Elger, Walter, Dr.**
**Schorlemer Allee 12 B**
**D-1000 Berlin 33 (DE)**
Erfinder : **Wiechert, Rudolf, Prof.-Dr.**
**Petzower Strasse 8 A**
**D-1000 Berlin 39 (DE)**
Erfinder : **Hofmeister, Helmut, Dr.**
**Weislingenstrasse 4**
**D-1000 Berlin 28 (DE)**
Erfinder : **Töpert, Michael, Dr.**
**Sigismundkorso 58**
**D-1000 Berlin 28 (DE)**

**EP 0 153 270 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 1α, 2α-Methylen-6-methylen- und 6α-methyl-pregnene der allgemeinen Formel I

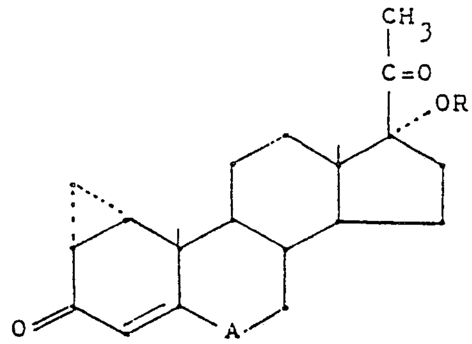

(I)

worin

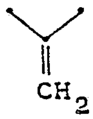

oder

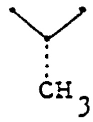

und

R ein Wasserstoffatom, eine Acyl- oder Alkoxymethylgruppe bedeuten, sowie Verfahren zu deren Herstellung und diese enthaltende pharmazeutische Präparate.

Die 6α-Methylpregnene der allgemeinen Formel I sind pharmakologisch wirksame Verbindungen. Sie hemmen die Lipogenese von Talgdrüsen bei epidermaler Applikation und sind im Antiandrogen-Test nach subcutaner und oraler Applikation nur schwach wirksam. Sie besitzen außerdem starke ovulationshemmende und schwache gestagene Wirksamkeit.

Die 6-Methylenverbindungen der allgemeinen Formel I, aus denen die 6α-Methylverbindungen hergestellt werden, zeigen bereits antiandrogene aber noch keine oder nur geringe ovulationshemmende Wirksamkeit.

Bei den freien 17-Hydroxyverbindungen der allgemeinen Formel I sind die gestagenen Eigenschaften weiter abgeschwächt, während die antiandrogenen Eigenschaften noch fortbestehen.

Die neuen Verbindungen der allgemeinen Formel I besitzen großes Interesse wegen der antiandrogenen Wirkung, die bei manchen Verbindungen mi einer ovulationshemmenden Wirkung gepaart ist. Sie sind insbesondere zur topischen Behandlung von Seborrhoe, Akne, Alopezie und Hirsutismus geeignet.

Das bislang wirksamste Antiandrogen mit ovulationshemmender (antigonadotroper) Wirkung ist das von Wiechert, Neumann et al. (Arzneimittelforschung 17 (1967), 1103) beschriebene Cyproteronacetat (17-Acetoxy-6-chlor-1α,2α-methylen-4,6-pregnadien-3,20-dion).

Der Wirkstoff Cyproteronacetat schränkt den Einfluß von Androgenen an ihren Erfolgsorganen durch kompetetive Hemmung weitgehend ein. So vermindert Cyproteronacetat bei Männern mit Sexualdeviationen die Triebstärke und stellt letztlich eine medikamentöse Alternative zur Kastration aus kriminologischen Erwägungen dar.

Von besonderer Bedeutung ist die Behandlung des Prostatakarzinoms und der Prostatahypertrophie mit Cyproteronacetat. Es ist allgemein bekannt, daß durch Androgene das Karzinom der Prostata — ebenso wie das Gewebe, auf dessen Boden es sich entwickelt — wachstumsfördernde Impulse erhält. Durch eine Ausschaltung der Androgenwirkung mit Hilfe von Antiandrogenen kann man das Karzinomwachstum hemmen.

Die antiandrogene Wirksamkeit von Cyproteronacetat ermöglicht eine spezifische Therapie von Androgenisierungserscheinungen bei Frauen : Die pathologische Behaarung bei Hirsutismus, die androgenetische Alopezie sowie die gesteigerte Talgdrüsenfunktion bei Akne und Seborrhö sind günstig beeinflußbar (Ärztliche Praxis 100 (1978) 3 461).

Cyproteronacetat wurde im Rahmen eines Syntheseprogramms für orale Gestagene synthetisiert. Die Verbindung zeigt eine starke progestative sowie antigonadotrope Wirkung. Die stark progestative Wirkung wurde im Clauberg-Test an Kaninchen und der antigonadotrope Effekt im Ovulationshemmtest an Ratten nachgewiesen.

Eine Kombination aus Cyproteronacetat (Gestagen) und Ethinylestradiol (Östrogen) steht als Präparat zur Behandlung von Androgenisierungserscheinungen bei der Frau und gleichzeitig als ein zuverlässiges Kontrazeptivum für solche Frauen zur Verfügung, die primär unter diesen Erscheinungen leiden oder bei denen Akne und ähnliche Symptome unter der Verwendung anderer Ovulationshemmer auftreten oder sich verschlimmern.

In allen diesen Fällen wird Cyproteronacetat systemisch angewandt. Dagegen führt die topische Anwendung von Cyproteronacetat zu keinem wesentlichen Erfolg.

Das pharmakologische Wirkungsspektrum der erfindungsgemäßen Verbindungen wurde am Beispiel von 17α-Acetoxy-6α-methyl-1α,2α-methylen-4-pregnen-3,20-dion im Vergleich mit Cyproteronacetat untersucht.

Zur Bestimmung der systemischen antiandrogenen Wirkung wurde die Hemmung der durch Testosteronpropionat bedingten Zunahme des Samenblasen- und Prostatagewichts von Ratten nach subcutaner und oraler Applikation ermittelt.

Im Ovulationshemmtest an der Ratte wurden die Testsubstanzen subcutan oder oral über 4 Tage verabfolgt und durch Tubeninspektion der %-Satz der Tiere ermittelt, bei denen die Ovulation gehemmt war.

Die Ergebnisse der gestagenen Wirkung wurden im Clauberg-Test nach subcutaner Applikation der Wirkstoffe an kastrierten weiblichen Kaninchen erhalten. In den histologischen Schnitten wurde die sekretorische Umwandlung des Endometriums bestimmt. Die Bestimmung erfolgte nach der McPhail-Skala (Beurteilungsgrade 1 - 4; 1 = keine Umwandlung; 4 = vollständige Umwandlung). Die Beeinflussung der Lipopenese durch die Testsubstanzen wird wie folgt ermittelt:

Männliche, fertile Hamster im Gewicht von etwa 100 bis 120 g werden kastriert und mit täglich 0,1 mg Testosteronpropionat subcutan substituiert. Das rechte Ohr jedes Versuchstieres wird zweimal täglich mit je 0,01 ml einer 1 %igen Lösung der Testsubstanz in Aceton (beziehungsweise bei der Kontrollgruppe nur mit 0,01 ml Lösungsmittel) drei Wochen lang behandelt. Dann werden die Tiere getötet und aus dem behandelten rechten Ohr sowie dem unbehandelten linken Ohr jeweils ein definiertes Gewebeareal von 8 mm Durchmesser ausgestanzt. Ventrale und dorsale Seite der Ausstanzungen werden entlang des Ohrknorpels voneinander getrennt, unverzüglich in Dulbecco's Modifikation of Eagle's Medium, dem 4 mMol Glutamin und 10 % Kälberserum zugesetzt war und das zur Vermeidung von mikrobieller Kontamination 100 IE/ml Penecillin, 100 μg/ml Streptomycin, 125 μg/ml Kanamycin, 25 IE/ml Nystatin und 10 μg/ml Gentamycin enthält, überführt und 1 Stunde lang bei 37 °C inkubiert.

Danach bringt man die Ausstanzungen unter sterilen Bedingungen in frisches Kulturmedium, das 1 μCi/ml $C^{14}$ markiertes Natriumacetat enthält und inkubiert 4 Stunden lang bei 37 °C. Anschließend gibt man die Proben in 2 ml einer Proteolyse-Lösung aus 0,05 Mol Tris-Puffer vom pH = 7,5, 0,01 Mol Dinatriumethylendiamintetraessigsäure, 0,5 % Natriumdodecylsulfat und 0,1 % Proteinase K (Firma E. Merck AG, Darmstadt, Bundesrepublik Deutschland), und inkubiert 24 Stunden lang bei 37 °C.

Die so erhaltenen Proben werden einmal mit 5 ml Chloroform und nochmals mit 3 ml Chloroform extrahiert, die vereinigten Chloroformextrakte im Vakuum eingeengt und ihr Gehalt an radiomarkierten Lipiden im Szintillationszähler ermittelt.

Die prozentuale Hemmung der Lipogenese der behandelten Kontrollgruppe wird durch Vergleich mit der nur mit Lösungsmittel behandelten Kontrollgruppe berechnet.

Die nachfolgende Tabelle zeigt die in diesen Tests erhaltenen Ergebnisse.

(Siehe Tabellen Seite 4 ff.)

Im Antiandrogen-Test an der Ratte ist die erfindungsgemäße 6α-Methylverbindung nach subcutaner und oraler Verabreichung bedeutend schwächer wirksam als Cyproteronacetat.

Im Ovulationshemmtest an der Ratte ist die erfindungsgemäße Verbindung dreimal stärker wirksam als Cyproteronacetat.

Im subcutanen Clauberg-Test am Kaninchen zeigt die 6α-Methylverbindung nur einen marginalen Effekt und ist wesentlich schwächer wirksam als Cyproteronacetat.

Überraschenderweise hemmt die erfindungsgemäße 6α-Methylverbindung bei epidermaler Applikation die Lipogenese von Talgdrüsen am Hamsterohr. Hierbei wird der Effekt von Testosteronpropionat auf die Stimulierung der Lipogenese bei kastrierten Tieren dosisabhängig aufgehoben. In der höchsten Dosierung von 1 % ist die Lipogenesehemmung der erfindungsgemäßen Verbindung ebenso stark ausgeprägt wie bei der kastrierten Kontrolle ohne Gabe von Testosteronpropionat, das heißt die Testosteronwirkung wird vollständig gehemmt.

Die 6α-Methylverbindungen der allgemeinen Formel I sind bevorzugt. In den 6-Methylenverbindungen der allgemeinen Formel I sind die günstigen Eigenschaften der daraus erhältlichen 6α-Methylverbindungen noch nicht so stark ausgeprägt.

Aufgrund der starken Lipogenesehemmung bei epidermaler Applikation und des geringen antiandrogenen Effekts nach subcutaner und oraler Applikation können insbesondere die 6α-Methylverbindungen der allgemeinen Formel I als Antiandrogene zur topischen Behandlung von Seborrhoe, Akne, Alopezie und Hirsutismus eingesetzt werden.

Die Herstellung der topischen Zubereitung erfolgt in üblicher Weise, indem man die Wirkstoffe mit geeigneten Trägerstoffen in die gewünschte Applikationsformen wie zum Beispiel Lösungen, Gels, Lotionen, Cremes, Salben, Puder oder Pflaster überführt.

Das Antiandrogen liegt in den pharmazeutischen Präparaten vorzugsweise in einer Konzentration von 0,05 bis 5,0 Gewichtsprozent vor.

Geeignete Trägerstoffe für Lösungen und Gels sind zum Beispiel Wasser, Ethanol, Propanol,

| Verbindung | Antiandrogen-Test s.c. Dosis /mg/ | s.c. Samenbl.* /mg/ | s.c. Prostata* /mg/ | p.o. Dosis /mg/ | p.o. Samenbl.* /mg/ | p.o. Prostata* /mg/ |
|---|---|---|---|---|---|---|
| 17α-Acetoxy-6α-methyl-1α,2α-methylen-4-pregnen-3,20-dion (gemäß Erfindung) | 1,0<br>0,3<br>0,1<br>0,03 | 26<br>27<br>33<br>27 | 20<br>20<br>29<br>19 | 3,0<br>1,0<br>0,3<br>0,1 | 36<br>44<br>57<br>57 | 33<br>39<br>43<br>48 |
| 17α-Acetoxy-6-chlor-1α,2α-methylen-4,6-pregnadien-3,20-dion (Cyproteronacetat als Vergleich | 3,0<br>1,0<br>0,3<br>0,1 | 7<br>12<br>16<br>29 | 8<br>13<br>14<br>25 | 10,0<br>3,0<br>1,0<br>0,3 | 9<br>14<br>18<br>38 | 11<br>13<br>14<br>29 |
| Kontrolle kastriert | | 6 | 7 | | 5 | 7 |
| Kontrolle kastriert + 0,1 mg Testosteronpropionat | | 37 | 32 | | 52 | 45 |

* Organgewicht pro 100 g Körpergewicht

| Verbindung | Ovulationshemmtest s.c. Dosis /mg/ | Ovulationshemmtest s.c. Hemmung % | Ovulationshemmtest p.o. Dosis /mg/ | Ovulationshemmtest p.o. Hemmung % | Clauberg-Test s.c. Dosis /mg/ | Clauberg-Test s.c. McPhail-Wert |
|---|---|---|---|---|---|---|
| 17α-Acetoxy-6α-methyl-1α,2α-methylen-4-pregnen-3,20-dion (gemäß Erfindung) | 1,0<br>0,3<br>0,1 | 100<br>83<br>33 | 1,0<br>0,3<br>0,1 | 100<br>100<br>0 | 0,03<br>0,01<br>0,003<br>0,001 | 1,6<br>1,5<br>1,3<br>1,3 |
| 17α-Acetoxy-6-chlor-1α,2α-methylen-4,6-pregnadien-3,20-dion (Cyproteronacetat als Vergleich | 3,0<br>1,0<br>0,3 | 100<br>60<br>16 | 3,0<br>1,0<br>0,3 | 100<br>100<br>0 | 0,1<br>0,03<br>0,01<br>0,003 | 2,9<br>2,7<br>2,3<br>1,6 |
| Kontrolle kastriert | | | | | | |
| Kontrolle kastriert + 0,1 mg Testosteronpropionat | | | | | | |

| Verbindung | Lipogenese-Test rechtes Ohr Konz. % | Lipogenese-Test rechtes Ohr Hemmung % | Lipogenese-Test linkes Ohr Konz. % | Lipogenese-Test linkes Ohr Hemmung % |
|---|---|---|---|---|
| 17α-Acetoxy-6α-methyl-1α,2α-methylen-4-pregnen-3,20-dion (gemäß Erfindung) | 1,0<br>0,3<br>0,1<br>0,03 | 66<br>40<br>48<br>26 | 1,0<br>0,3<br>0,1<br>0,03 | 22<br>5<br>11<br>9 |
| 17α-Acetoxy-6-chlor-1α,2α-methylen-4,6-pregnadien-3,20-dion (Cyproteronacetat als Vergleich | 1,0 | 29 | 1,0 | 28 |
| Kontrolle kastriert | | 62 | | 61 |
| Kontrolle kastriert + 0,1 mg Testosteron-propionat | | 0 | | 12 |

Glycerin, Methylcellulose, Hydroxypropylcellulose, Carboxypolymethylen usw.

Die Lotionen oder Cremes (Öl/Wasser-Emulsionen) und die Salben (Wasser/Öl-Emulsionen) können in konventioneller Weise unter Verwendung konventioneller Emulgatoren hergestellt werden (Kirk Othmer : Enzyclopedia of Chemical Technology, 3. Auflage, 1979 ; John Wiley & Sons, New York, Vol. 8, Seiten 900 - 930, und Dr. Otto-Albrecht Neumüller : Römpps Chemie Lexikon, 7. Auflage, 1973 ; Franckh'sche Verlagshandlung Stuttgart, Seiten 1 009 - 1 013). Die für diese Emulsionen verwendeten Wachse, Emulgatoren und übrigen Zusätze sind die gleichen, wie man sie konventionellerweise verwendet (Dr. Otto-Albrecht Neumüller : Römpps Chemie Lexikon, 7. Auflage, 1973 ; Franckh'sche Verlagshandlung Stuttgart, Seiten 1 427 und 1 428).

Die erfindungsgemäße topische Zubereitung in Form einer Öl/Wasser-Emulsion kann aus hydrophilen und/oder lipophilen Wirkstoffen, Fettphase, Öl/Wasser-Emulgator, wäßriger Phase und Konservierungsmittel bestehen.

Als hydrophile und/oder lipophile Zusätze können Feuchthaltefaktoren (Hydrokomplexe), wie zum Beispiel Glycerin, Polyäthylenglykole oder Aminosäuregemische, Puroba-Oil (= Jojoba-Öl), Vitamine (vorzugsweise Vitamin A und E), Vitalkomplexe (wie zum Beispiel Placentaextrakte), Enzyme, Kräuterauszüge (wie zum Beispiel Hammamelis Extrakt oder Kamillenextrakt) oder Proteine (wie zum Beispiel Collagen) eingesetzt werden. Als ölige Phase oder als Fettphase in der Öl/Wasser-Emulsion eignen sich Kohlenwasserstoffe, wie zum Beispiel Vaseline, Paraffine oder Stearin, oder Wachse, wie zum Beispiel Bienenwachs. Geeignete Öl/Wasser-Emulgatoren sind beispielsweise Stearylalkohol, Polyoxyäthylenstearate (wie zum Beispiel MYRJ(R)), Komplexemulgatoren (wie zum Beispiel Amphoterin(R)) und Sorbitanfettsäureester (wie zum Beispiel Span(R)) oder Carboxyvinylpolymerisate (wie zum Beispiel Carbopol(R)). Die wäßrige Phase kann zusätzlich noch Puffersubstanzen, wie zum Beispiel das Dinatriumsalz oder Äthylendiamin-N,N,N', N'-tetraessigsäure und Konservierungsmittel, wie Chlorquinaldol, Parabene oder Benzalkoniumchlorid, enthalten.

In der Öl/Wasser-Emulsion beträgt der Anteil der inneren Emulsion vorzugsweise 10 bis 49 Gewichtsprozent, die Teilchengröße der inneren Emulsion liegt vorzugsweise zwischen 1 $\mu$ und 100 $\mu$.

Die erfindungsgemäße topische Zubereitung in Form einer Wasser/Öl-Emulsion besteht ebenfalls aus hydrophilen und/oder lipophilen Zusätzen, wie sie auch in der Öl/Wasser-Emulsion verwendet werden, Fettphase, Wasser/Öl-Emulgator und wäßriger Phase. Als ölige Phase oder Fettphase der Wasser/Öl-Emulsion können Kohlenwasserstoffe, zum Beispiel Paraffine und Vaseline, synthetische, pflanzliche und tierische Öle bzw. Wachse (wie zum Beispiel Olivenöl, Erdnußöl, feines Knochenöl, Mandelöl, Lanolin, Bienenwachs oder Sonnenblumenöl) eingesetzt werden, als wäßrige Phase gereinigtes demineralisiertes Wasser und als Wasser/Öl-Emulgator Wollfett (= Lanolin), Fettalkohole, wie zum Beispiel Cetylalkohol, Myristylalkohol, Stearylalkohol oder Cerylalkohol, Fettsäureester, wie zum Beispiel Bienenwachs (Cera alba) oder Wachsalkoholester oder Mischester (wie zum Beispiel Dehymuls(R)).

In der Wasser/Öl-Emulsion beträgt der Anteil der inneren Emulsion vorzugsweise 30 bis 49 Gewichtsprozent, die Teilchengröße der inneren Emulsion liegt vorzugsweise zwischen 1 $\mu$ und 100 $\mu$.

Das feindisperse System wird zusätzlich mit dem mikronisierten Wirkstoff (Korngröße vorzugsweise 1 bis 20 $\mu$) und gegebenenfalls noch mit Duftstoffen, wie zum Beispiel diejenige der Crematest(R)-Serie, versetzt und bis zur gleichmäßigen Verteilung derselben gerührt.

Die erfindungsgemäßen Verbindungen der allgemeinen Formel I besitzen in 17$\alpha$-Stellung des Steroidgerüstes ein Hydroxy-, Acyloxy- oder Alkoxymethoxygruppe (OR).

Unter Acyloxy sind solche Säurereste zu verstehen, die sich von Säuren ableiten, die in der Steroidchemie üblicherweise für Veresterungen angewendet werden. Bevorzugte Säuren sind Carbonsäuren mit 1 bis 7 Kohlenstoffatomen, zum Beispiel Monocarbonsäuren wie Essigsäure, Propionsäure, Buttersäure, Capronsäure, Önanthsäure und Benzoesäure, und Dicarbonsäuren wie Bernsteinsäure und Adipinsäure.

Unter Alkoxy in Alkoxymethoxy sind vorzugsweise Methoxy-, Ethoxy- und Propoxy zu verstehen.

Die Herstellung der erfindungsgemäßen Verbindungen der allgemeinen Formel I erfolgt nach Anspruch 7.

Der Ringschluß der 1$\alpha$-Chlormethylgruppe unter HCl-Abspaltung zur 1$\alpha$,2$\alpha$-Methylengruppe erfolgt mit starken Basen.

Als Basen kommen sowohl anorganische, wie Natrium- oder Kaliumhydroxid, als auch organische Basen, wie Collidin, Lutidin, Pyridin usw. infrage. Bei Verwendung anorganischer Basen wird die Chlorwasserstoffabspaltung zweckmäßigerweise in alkoholischer Lösung vorgenommen. Als Reaktionstemperaturen sind vorzugsweise die Siedetemperaturen der betreffenden Lösungsmittel bzw. organischen Basen geeignet. Der gewünschte Ringschluß zum 1,2-Methylen kann aber auch durch bloße Filtration der in einem organischen Lösungsmittel gelösten 1-Chlormethylverbindung über Aluminiumoxid erfolgen, wobei die Anwendung erhöhter Temperaturen überflüssig wird.

Die Hydrierung der 6-Methylenverbindung erfolgt durch katalytische Wasserstoff-Transferreaktion (Kontakte (Merck) 1 (80) 3 - 10). Durch saure Aufarbeitung wird die Ausbildung der 6$\alpha$-Methylgruppe begünstigt.

HCl-Abspaltung zur 1,2-Methylen- und Hydrierung zur 6-Methylverbindung können auch in einem Schritt unter den Bedingungen der katalytischen Wasserstoff-Transferreaktion durchgeführt werden. Nach einer bevorzugten Ausführungsform wird die 1$\alpha$-Chlormethyl-6-methylen-Verbindung der allgemeinen Formel II mit Cyclohexen in Gegenwart von Palladium auf Kohle der Transferhydrierung unterworfen.

Die sich gegebenenfalls anschließende Verseifung der 17α-Acyloxygruppe wird nach bekannten Methoden mit Alkali durchgeführt.

Die Herstellung der Ausgangsverbindungen der allgemeinen Formel II sei anhand des folgenden Reaktionsschemas erläutert :

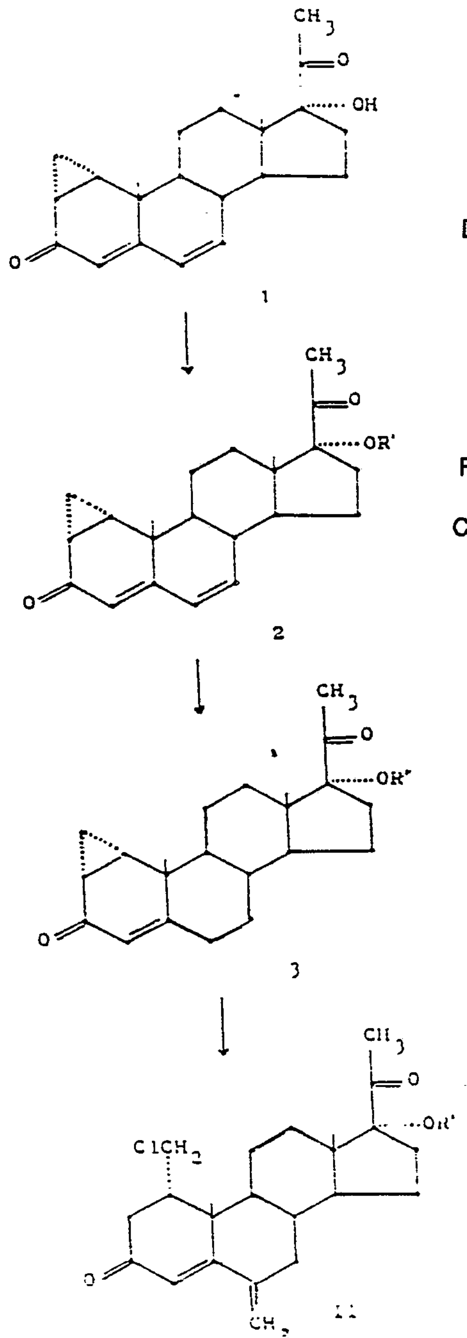

1

DE-AS 1 189 991

2

R' = $COCH_3$

Chem. Ber. *93* (1960), 1710

3

R' = $—COCH_3$

USP 3 652 604

Herstellung der 17α-Acyloxy- und Methoxymethoxy-1α,2α-methylen-4,6-pregnadien-3,20-dione (2) aus der entsprechenden 17α-Hydroxyverbindung (1)

a) 1α,2α-Methylen-17α-propionyloxy-4,6-pregnadien-3,20-dion

Eine Suspension von 7,1 g 17α-Hydroxy-1α,2α-methylen-4,6-pregnadien-3,20-dion (DE-AS-1 189 991) in 114 ml Propionsäure wird bei 0 °C tropfenweise mit 43 ml Trifluoressigsäureanhydrid versetzt. Man rührt 4 Stunden bei Raumtemperatur weiter und arbeitet nach der Eiswasser-Kochsalz-Fällung wie üblich auf. Man chromatographiert das Rohprodukt an 600 g Kieselgel mit einem Hexan-Aceton-Gradienten (0 - 20 % Aceton) und isoliert 5,8 g 1α,2α-Methylen-17α-propionyloxy-4,6-pregnadien-3,20-dion.

Unter analogen Bedingungen werden hergestellt :

b) 17α-Butyryloxy-1α,2α-methylen-4,6-pregnadien-3,30-dion ;

c) 1α,2α-Methylen-17α-valeryloxy-4,6-pregnadien-3,20-dion.

d) 17α-Hexanoyloxy-1α,2α-methylen-4,6-pregnadien-3,20-dion

10,0 g 17α-Hydroxy-1α,2α-methylen-4,6-pregnadien-3,20-dion werden in 200 ml Capronsäureanhydrid mit 5,0 g p-Toluolsulfonsäure unter Stickstoff 24 Stunden bei Raumtemperatur gerührt. Das überschüssige Capronsäureanhydrid wird in Gegenwart von 50 ml Pyridin mit Wasserdampf abdestilliert. Der Rückstand wird mit Methylenchlorid extrahiert, der Extrakt mit Natriumsulfat getrocknet und im Vakuum eingeengt. Das Rohprodukt wird an Kieselgel mit einem Aceton-Pentan-Gradienten gereinigt. Man erhält 11,3 g 17α-Hexanoyloxy-1α,2α-methylen-4,6-pregnadien-3,20-dion. $[\alpha]_D^{25} = + 85°$.

e) 17α-Methoxymethoxy-1α,2α-methylen-4,6-pregnadien-3,20-dion

Eine Lösung von 4,0 g 17α-Hydroxy-1α,2α-methylen-4,6-pregnadien-3,20-dion in 28 ml wasserfreiem Methylenchlorid und 18 ml Formaldehyddimethylacetal wird portionsweise mit einem Gemisch aus 6,0 g Kieselgur W 20 und 3,0 g Phosphorpentoxid versetzt. Man rührt 45 Minuten bei Raumtemperatur nach, saugt ab und eluiert den Rückstand mehrmals mit Methylenchlorid. Das Rohprodukt wird an 750 g Kieselgel mit einem Hexan-Aceton-Gradienten gereinigt.
Ausbeute : 3,1 g 17α-Methoxymethoxy-1α,2α-methylen-4,6-pregnadien-3,20-dion.

Herstellung der 17α-Acyloxy- und Methoxymethoxy-1α,2α-methylen-4-pregnen-3,20-dione (3) aus den entsprechenden 4,6-Pregnadienen (2)

a) 17α-Acetoxy-1α,2α-methylen-4-pregnen-3,20-dion

Eine Lösung von 60,0 g 17α-Acetoxy-1α,2α-methylen-4,6-pregnadien-3,20-dion [R. Wiechert et al. Chem. Ber. 93, 1 710 (1960)] in 1,25 l Dimethylformamid wird mit 6,3 g 10 %igem $Pd/CaCO_3$ versetzt und anschließend hydriert. Nach einer Wasserstoffaufnahme von 5,1 l wird der Katalysator abfiltriert und die Reaktionslösung in Eiswasser eingerührt. Man saugt den Niederschlag ab, arbeitet wie üblich auf und reinigt das Rohprodukt an 1,5 kg Kieselgel mit einem Pentan-Aceton-Gradienten (0 - 30 % Aceton).
Ausbeute : 34,3 g 17α-Acetoxy-1α,2α-methylen-4-pregnen-3,20-dion.
Schmelzpunkt : 268 - 270°C. $[\alpha]_D = + 215°$.

b) 1α,2α-Methylen-17α-propionyloxy-4-pregnen-3,20-dion

Ein Gemisch aus 2 ml Cyclohexen und 5 ml Ethanol wird mit 50 mg 10 %iger Pd/Aktivkohle 1 Stunde bei 80 °C Badtemperatur gerührt. Nach der Zugabe von 500 mg 1α,2α-Methylen-17α-propionyloxy-4,6-pregnadien-3,20-dion wird 1,5 Stunden bei 80 °C weitergerührt. Anschließend wird der Katalysator abfiltriert und das Filtrat im Vakuum zur Trockne eingeengt. Das Rohprodukt wird an 50 g Kieselgel mit einem Hexan-Aceton-Gradienten chromatographiert. Man isoliert 380 mg 1α,2α-Methylen-17α-propionyloxy-4-pregnen-3,20-dion.

c) 17α-Butyryloxy-1α,2α-methylen-4-pregnen-3,20-dion

Eine Lösung von 3,0 g 17α-Butyryloxy-1α,2α-methylen-4,6-pregnadien-3,20-dion in einem Gemisch aus 225 ml Tetrahydrofuran und 75 ml Methanol wird mit 2,7 g Tristriphenylphosphin-rhodium-i-chlorid 6,5 Stunden hydriert (DC- und UV-Kontrolle). Man engt im Vakuum zur Trockne ein und reinigt das Rohprodukt an 300 g Kieselgel mit einem Hexan-Aceton-Gradienten.
Ausbeute : 2,1 g 17α-Buryryloxy-1α,2α-methylen-4-pregnen-3,20-dion.
Unter den Hydrierbedingungen a) - c) werden aus 2 c) - 2 e) hergestellt :

d) 1α,2α-Methylen-17α-valeryloxy-4-pregnen-3,20-dion ;

e) 17α-Hexanoyloxy-1α,2α-methylen-4-pregnen-3,20-dion ;

f) 17α-Methoxymethoxy-1α,2α-methylen-4-pregnen-3,20-dion.

Herstellung der 1α-Chlormethyl-6-methylen-pregnene der allgemeinen Formel II aus den entsprechenden 6-Desmethylen-1α,2α-methylen-pregnenen (3)

a) 17α-Acetoxy-1α-chlormethyl-6-methylen-4-pregnen-3,20-dion

Eine Suspension von 9,0 g Natriumacetat in 270 ml Chloroform, 270 ml Formaldehyddimethylacetal

und 35 ml Phosphoroxychlorid wird mit 9,0 g 17α-Acetoxy-1α-2α-methylen-4-pregnen-3,20-dion 5 Stunden bei 65 °C Badtemperatur gerührt. Die Reaktionslösung wird mit einer gesättigten Natriumcarbonatlösung neutralisiert und mit Methylenchlorid verdünnt. Die organische Phase wird abgetrennt und wie üblich aufgearbeitet. Das Rohprodukt wird an 500 g Kieselgel mit einem Hexan-Essigester-Gradienten (0 - 30 % Essigester) gereinigt. Man isoliert 7,5 g 17α-Acetoxy-1α-chlormethyl-6-methylen-4-pregnen-3,20-dion vom Schmelzpunkt 190 - 192 °C.

Unter analogen Reaktionsbedingungen erhält man aus 3 b) - 3 f) :

b) 1-Chlormethyl-6-methylen-17α-propionyloxy-4-pregnen-3,20-dion ;

c) 17α-Buryryloxy-1-chlormethyl-6-methylen-4-pregnen-3,20-dion ;

d) 1-Chlormethyl-6-methylen-17α-valeryloxy-4-pregnen-3,20-dion ;

e) 1-Chlormethyl-17α-hexanoyloxy-6-methylen-4-pregnen-3,20-dion ;

f) 1-Chlormethyl-17α-methoxymethoxy-6-methylen-4-pregnen-3,20-dion

## Beispiel 1

a) 17α-Acetoxy-1α,2α-methylen-6-methylen-4-pregnen-3,20-dion

Eine Lösung von 7,5 g 17α-Acetoxy-1-chlor-methyl-6-methylen-4-pregnen-3,20-dion in 66 ml γ-Collidin wird 1 Stunde bei 180 °C Badtemperatur gerührt und anschließend auf eine Eiswasser-Kochsalz-Lösung gegeben. Man filtriert ab und arbeitet den Rückstand wie üblich auf. Das Rohprodukt wird an 700 g Kieselgel mit einem Hexan-Essigester-Gradienten gereinigt.

Ausbeute : 4,3 g 17α-Acetoxy-1α,2α-methylen-6-methylen-4-pregnen-3,20-dion vom Schmelzpunkt 205 - 206 °C.

Unter analogen Reaktionsbedingungen erhält man aus den entsprechenden Verbindungen der allgemeinen Formel II :

b) 1α,2α-Methylen-6-methylen-17α-propionyloxy-4-pregnen-3,20-dion ;

c) 17α-Butyryloxy-1α,2α-methylen-6-methylen-4-pregnen-3,20-dion ;

d) 1α,2α-Methylen-6-methylen-17α-valeryloxy-4-pregnen-3,20-dion ;

e) 17α-Hexanoyloxy-1α,2α-methylen-6-methylen-4-pregnen-3,20-dion ;

f) 17α-Methoxymethoxy-1α,2α-methylen-6-methylen-4-pregnen-3,20-dion.

## Beispiel 2

17α-Hydroxy-1α,2α-methylen-6-methylen-4-pregnen-3,20-dion

500 mg 17α-Acetoxy-1α,2α-methylen-6-methylen-4-pregnen-3,20-dion werden in 30 ml Methanol und 16 ml 5 %iger Natronlauge 16 Stunden unter Argon gerührt. Es wird auf eine Eiswasser-Kochsalz-Lösung gegeben und wie üblich aufgearbeitet. Das Rohprodukt wird an 50 g Kieselgel mit einem Hexan-Aceton-Gradienten gereinigt. Man isoliert 380 mg 17α-Hydroxy-1α,2α-methylen-6-methylen-4-pregnen-3,20-dion.

## Beispiel 3

a) 17α-Acetoxy-6α-methyl-1α,2α-methylen-4-pregnen-3,20-dion

Eine Suspension von 533 mg 10 %igen Pd/C in 31 ml Cyclohexen und 46 ml Ethanol wird 1 Stunde bei 80 °C Badtemperatur gerührt. Man fügt 7,5 g 17-Acetoxy-1-chlormethyl-6-methylen-4-pregnen-3,20-dion hinzu und rührt 18 Stunden bei 80 °C nach. Anschließend wird der Katalysator abgesaugt, mit warmem Ethanol gewaschen und das Filtrat mit 7 ml konzentrierter Salzsäure versetzt. Man engt auf 1/3 des Reaktionsvolumens ein und gibt auf eine Eiswasser-Kochsalz-Lösung. Nach der üblichen Aufarbeitung wird das Rohprodukt an 700 g Kieselgel mit einem Hexan-Essigester-Gradienten gereinigt.

Ausbeute 4,3 g 17α-Acetoxy-6α-methyl-1α,2α-methylen-4-pregnen-3,20-dion vom Schmelzpunkt 198 - 200 °C.

Unter analogen Reaktionsbedingungen erhält man aus den entsprechenden Verbindungen II :

b) 6α-Methyl-1α,2α-methylen-17α-propionyloxy-4-pregnen-3,20-dion ;

c) 17α-Buryryloxy-6α-methyl-1α,2α-methylen-4-pregnen-3,20-dion ;

d) 6α-Methyl-1α,2α-methylen-17α-valeryloxy-4-pregnen-3,20-dion ;

e) 17α-Hexanoyloxy-6α-methyl-1α,2α-methylen-4-pregnen-3,20-dion ;

f) 17α-Methoxymethoxy-6α-methyl-1α,2α-methylen-4-pregnen-3,20-dion ;

g) 17α-Hydroxy-6α-methyl-1α,2α-methylen-4-pregnen-3,20-dion.

Unter den Bedingungen des Beispiels 2 werden 450 mg 17α-Acetoxy-6α-methyl-1α,2α-methylen-4-pregnen-3,20-dion zu 310 mg 17α-Hydroxy-6α-methyl-1α,2α-methylen-4-pregnen-3,20-dion umgesetzt, aufgearbeitet und gereinigt. Man erhält die Titelverbindung.
Beispiele für die Herstellung topischer Zubereitungen

Beispiel 4

a) Herstellung der Öl/Wasser-Emulsion

10,000 g Dinatriumedetat und 10,000 g Chlorquinaldol werden in 300,000 g gereinigtem demineralisiertem Wasser gelöst und mit 10,000 g Carbopol(R) versetzt.
Diese Mischung wird unter kräftigem Rühren in eine Schmelze von 80,000 g Vaseline (DAB 8) — DAB ist die Abkürzung dür das Deutsche Arzneibuch, amtliche Ausgabe, 8. Auflage, 1978 — 40,000 g Stearylalkohol, 30,000 g MYRJ(R) und 50,000 g Pur-oba-Öl eingetragen. Man rührt die Mischung noch so lange, bis eine Emulsion mit einer Teilchengröße von 20 - 70 μm entsteht.

b) Herstellung der Wasser/Öl-Emulsion

230,000 g gereinigtes demineralisiertes Wasser werden unter kräftigem Rühren in eine Schmelze von 220,000 g Vaseline (DAB 8), 10,000 g Dehymuls(R) und 10,000 g Bienenwachs eingetragen. Man rührt die Mischung noch so lange, bis eine Emulsion mit einer Teilchengröße von 20 - 70 μm entsteht.

c) Herstellung einer Creme

Die Wasser/Öl-Emulsion wird unter kräftigem Rühren unter einem Vakuum von 13,33 mbar (10 torr) in die Öl/Wasser-Emulsion eingetragen. Man rührt noch so lange, bis eine Dispersion mit einer Teilchengröße von 10 bis 50 μm entsteht, und entfernt das Vakuum.
In 98,000 g dieser Salbengrundlage setzt man unter Rühren 2,000 g 17α-Acetoxy-6α-methyl-1α,2α-methylen-4-pregnen-3,20-dion — mikronisiert ; Teilchengröße vorwiegend 1 bis 20 μm — hinzu und rührt, bis sich der Wirkstoff in der Salbengrundlage gleichmäßig verteilt hat.

Beispiel 5

99,000 g der gemäß Beispiel 4 c) hergestellten Salbengrundlage werden mit 1,000 g 17α-Acetoxy-6α-methyl-1α,2α-methylen-4-pregnen-3,20-dion — mikronisiert ; Teilchengröße vorwiegend 1 bis 20 μm — versetzt und gerührt, bis sich der Wirkstoff in der Salbengrundlage gleichmäßig verteilt.

Beispiel 6

Zusammensetzung einer öligen Lösung zur Behandlung schwerer Androgenisierungserscheinungen

Pro Ampulle zu 1 ml werden
50 mg 17α-Acetoxy-6α-methyl-1α,2α-methylen-4-pregnen-3,20-dion in
403,4 mg Rizinusöl und
618,6 mg Benzylbenzoat gelöst, sterilisiert und abgefüllt.

**Patentansprüche** (für die Vertragsstaaten : BE, CH, LI, DE, FR, GB, IT, LU, NL, SE)

1. 1α,2α-Methylen-6-methylen- und 6α-methyl-pregnene der allgemeinen Formel I

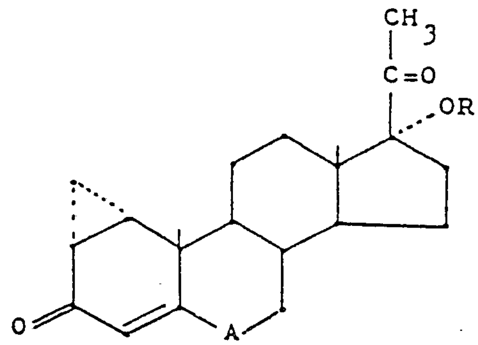

(I)

worin

A

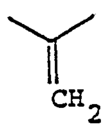 , oder 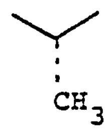 und

R ein Wasserstoffatom, eine Acyl- oder Alkoxymethylgruppe bedeuten.

2. 17α-Acetoxy-1α,2α-methylen-6-methylen-4-pregnen-3,20-dion,
1α,2α-Methylen-6-methylen-17α-propionyloxy-4-pregnen-3,20-dion,
17α-Butyryloxy-1α,2α-methylen-6-methylen-4-pregnen-3,20-dion,
1α,2α-Methylen-6-methylen-17α-valeryloxy-4-pregnen-3,20-dion,
17α-Hexanoyloxy-1α,2α-methylen-6-methylen-4-pregnen-3,20-dion,
17α-Methoxymethoxy-1α,2α-methylen-6-methylen-4-pregnen-3,20-dion.

3. 17α-Hydroxy-1α,2α-methylen-6-methylen-4-pregnen-3,20-dion.

4. 17α-Acetoxy-6α-methyl-1α,2α-methylen-4-pregnen-3,20-dion,
6α-Methyl-1α,2α-methylen-17α-propionyloxy-4-pregnen-3,20-dion,
17α-Butyryloxy-6α-methyl-1α,2α-methylen-4-pregnen-3,20-dion,
6α-Methyl-1α,2α-methylen-17α-valeryloxy-4-pregnen-3,20-dion,
17α-Hexanoyloxy-6α-methyl-1α,2α-methylen-4-pregnen-3,20-dion,
17α-Methoxymethoxy-6α-methyl-1α,2α-methylen-4-pregnen-3,20-dion.

5. 17α-Hydroxy-6α-methyl-1α,2α-methylen-4-pregnen-3,20-dion.

6. Pharmazeutische Präparate, gekennzeichnet durch den Gehalt an Verbindungen gemäß Anspruch 1. bis 5.

7. Verfahren zur Herstellung von 1α,2α-Methylen-6-methylen- und 6α-methyl-pregnenen der allgemeinen Formel I

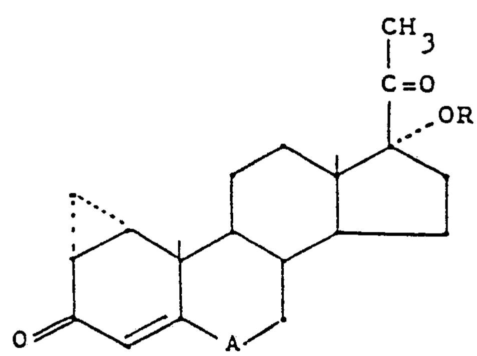

(I)

worin

A

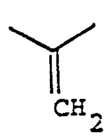 , oder 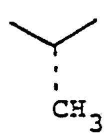 und

R ein Wasserstoffatom, eine Acyl- oder Alkoxymethylgruppe

bedeuten, dadurch gekennzeichnet, daß man 1α-Chlormethyl-6-methylen-pregnene der allgemeinen Formel II

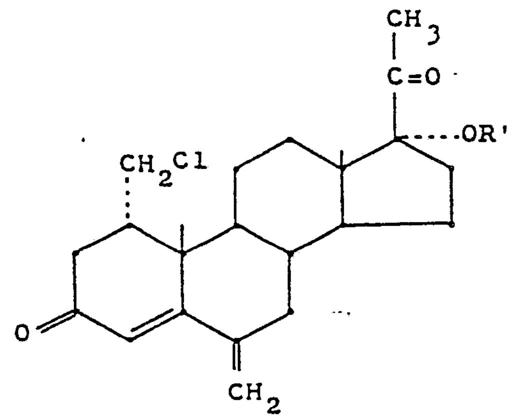

(II)

worin R' eine Acyl- oder Alkoxymethylgruppe darstellt, mit einer Base behandelt, wobei durch HCl-Abspaltung der 1,2-Methylenring gebildet wird, und gegebenenfalls anschließend die 6-Methylenverbindung durch katalytische Wasserstoff-Transferreaktion und saure Aufarbeitung in die 6α-Methylverbindung überführt oder HCl-Abspaltung zur 1,2-Methylen- und Hydrierung zur 6α-Methylverbindung unter den Bedingungen der katalytischen Wasserstoff-Transferreaktion mit saurer Aufarbeitung in einem Schritt durchführt und gegebenenfalls anschließend eine 17α-Acyloxygruppe hydrolysiert.

**Patentanspruch** (für den Vertragsstaat AT)

Verfahren zur Herstellung von 1α,2α-Methylen-6-methylen- und 6α-methyl-pregnenen der allgemeinen Formel I

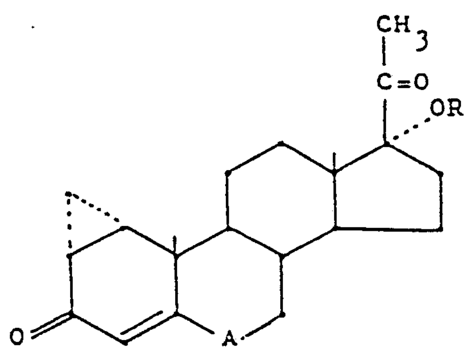

(I)

worin

A 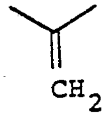 oder 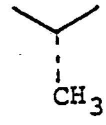 und

R ein Wasserstoffatom, eine Acyl- oder Alkoxymethylgruppe bedeuten, dadurch gekennzeichnet, daß man 1α-Chlormethyl-6-methylen-pregnene der allgemeinen Formel II

(Siehe Formel Seite 14 f.)

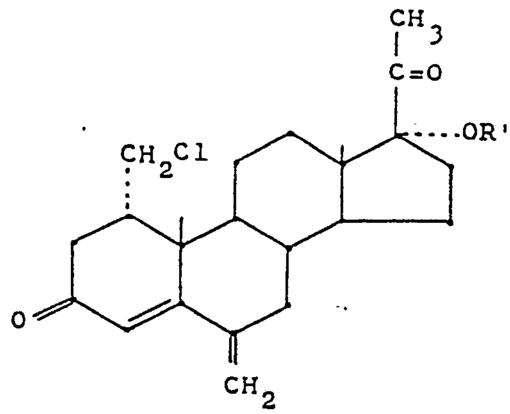

(II)

worin R' eine Acyl- oder Alkoxymethylgruppe darstellt, mit einer Base behandelt, wobei durch HCl-Abspaltung der 1,2-Methylenring gebildet wird, und gegebenenfalls anschließend die 6-Methylenverbindung durch katalytische Wasserstoff-Transferreaktion und saure Aufarbeitung in die 6α-Methylverbindung überführt oder HCl-Abspaltung zur 1,2-Methylen- und Hydrierung zur 6α-Methylverbindung unter den Bedingungen der katalytischen Wasserstoff-Transferreaktion mit saurer Aufarbeitung in einem Schritt durchführt und gegebenenfalls anschließend eine 17α-Acyloxygruppe hydrolysiert.

**Claims** (for the Contracting States : BE, CH, DE, FR, GB, IT, LU, NL, SE)

1. 1α,2α-Methylene-6-methylene- and 6α-methyl-pregnenes of general Formula I

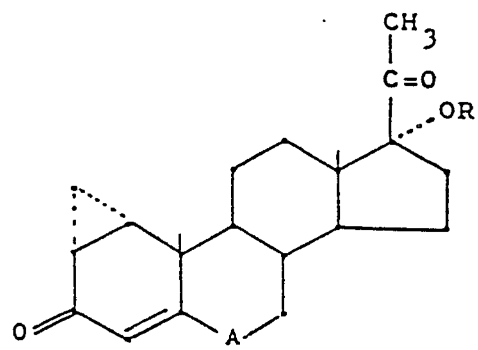

(I)

wherein

A is 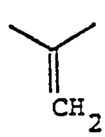 , or 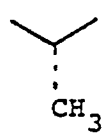 and

R is a hydrogen atom, an acyl group, or an alkoxymethyl group.

2. 17α-Acetoxy-1α,2α-methylene-6-methylene-4-pregnene-3,20-dione, 1α,2α-methylene-6-methylene-17α-propionyloxy-4-pregnene-3,20-dione, 17α-butyryloxy-1α,2α-methylene-6-methylene-4-pregnene-3,20-dione, 1α,2α-methylene-6-methylene-17α-valeryloxy-4-pregnene-3,20-dione, 17α-hexanoyloxy-1α,2α-methylene-6-methylene-4-pregnene-3,20-dione, 17α-methoxymethoxy-1α,2α-methylene-6-methylene-4-pregnene-3,20-dione.

3. 17α-Hydroxy-1α,2α-methylene-6-methylene-4-pregnene-3,20-dione.

4. 17α-Acetoxy-6α-methyl-1α,2α-methylene-4-pregnene-3,20-dione, 6α-methyl-1α,2α-methylene-17α-propionyloxy-4-pregnene-3,20-dione, 17α-butyryloxy-6α-methyl-1α,2α-methylene-4-pregnene-3,20-dione, 6α-methyl-1α,2α-methylene-17α-valeryloxy-4-pregnene-3,20-dione, 17α-hexanoyloxy-6α-methyl-1α,2α-methylene-4-pregnene-3,20-dione, 17α-methoxymethoxy-6α-methyl-1α,2α-methylene-4-pregnene-3,20-dione.

5. 17α-Hydroxy-6α-methyl-1α,2α-methylene-4-pregnene-3,20-dione.

6. Pharmaceutical preparations characterized in that they contain compounds according to claims 1-5.

7. Process for the preparation of 1α,2α-methylene-6-methylene- and 6α-methylpregnenes of general Formula I

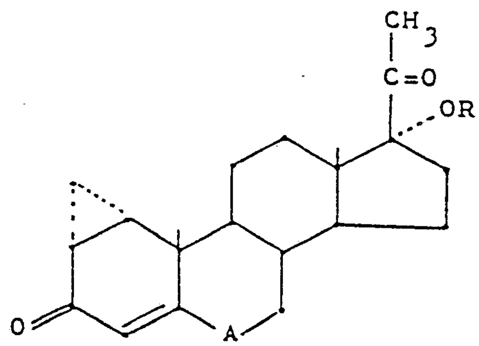

(I)

wherein

A is

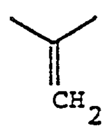

, or

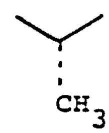

and

R is a hydrogen atom, an acyl group, or an alkoxymethyl group,
characterized by treating 1α-chloromethyl-6-methylene-pregnenes of general Formula II

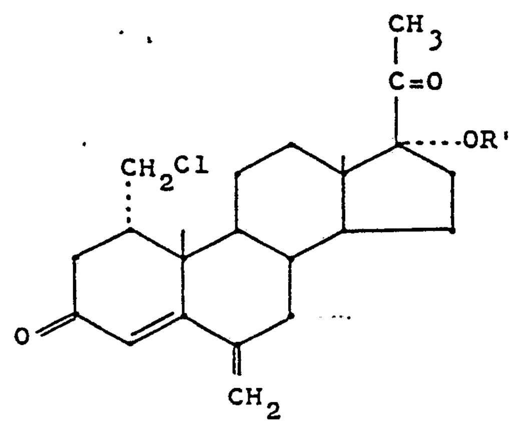

(II)

wherein R′ is an acyl or alkoxymethyl group,
with a base, thus forming the 1,2-methylene ring by splitting off HCl, and optionally thereafter converting the 6-methylene compound into the 6α-methyl compound by catalytic hydrogen transfer reaction and acidic working-up process, or performing in one step HCl cleavage to the 1,2-methylene compound and hydrogenation to the 6α-methyl compound under the conditions of catalytic hydrogen transfer reaction with an acidic working-up step, and optionally subsequently hydrolyzing a 17α-acyloxy group.

**Claim** (for the Contracting State AT)

Process for the preparation of 1α,2α-methylene-6-methylene- and 6α-methylpregnenes of general Formula I

(See Formula page 16)

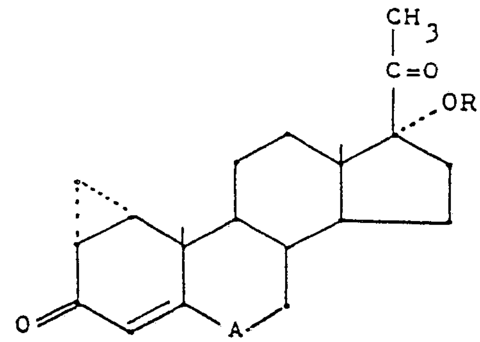

(I)

wherein

A is 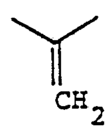 or 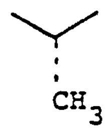 and

R is a hydrogen atom, an acyl group, or an alkoxymethyl group, characterized by treating 1α-chloromethyl-6-methylene-pregnenes of general Formula II

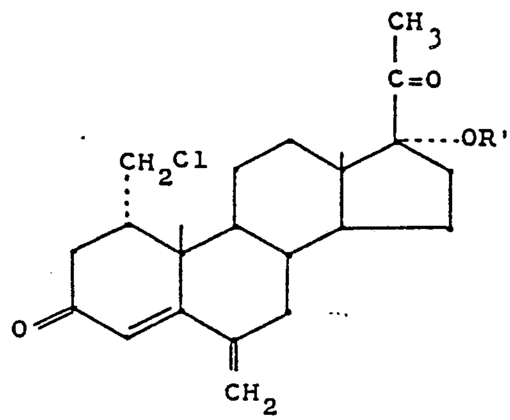

(II)

wherein R′ is an acyl or alkoxymethyl group, with a base, thus forming the 1,2-methylene ring by splitting off HCl, and optionally thereafter converting the 6-methylene compound into the 6α-methyl compound by catalytic hydrogen transfer reaction and acidic working-up process, or performing in one step HCl cleavage to the 1,2-methylene compound and hydrogenation to the 6α-methyl compound under the conditions of catalytic hydrogen transfer reaction with an acidic working-up step, and optionally subsequently hydrolyzing a 17α-acyloxy group.

**Revendications** (pour les Etats contractants : BE, CH, DE, FR, GB, IT, LI, LU, NL, SE)

1. 1α,2α-méthylène-6-méthylène-prégnènes et 1α,2α-méthylène-6α-méthyl-prégnènes répondant à la formule générale I :

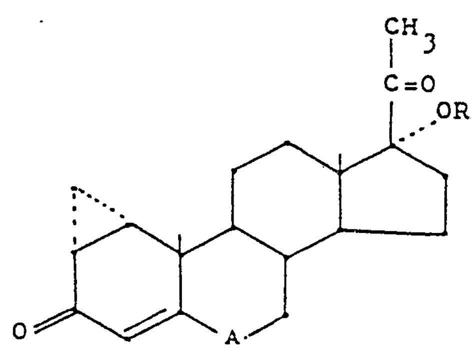

(I)

dans laquelle A représente un groupement

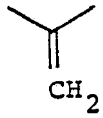 ou 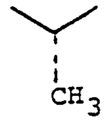

et R représente un atome d'hydrogène ou un groupe acyle ou alcoxyméthyle.

2. La 17α-acétoxy-1α,2α-méthylène-6-méthylène-4-prégnène-3,20-dione, la 1α,2α-méthylène-6-méthylène-17α-propionyloxy-4-prégnène-3,20-dione, la 17α-butyryloxy-1α,2α-méthylène-6-méthylène-4-prégnène-3,20-dione, la 1α,2α-méthylène-6-méthylène-17α-valéryloxy-4-prégnène-3,20-dione, la 17α-hexanoyloxy-1α,2α-méthylène-6-méthylène-4-prégnène-3,20-dione, la 17α-méthoxyméthoxy-1α,2α-méthylène-6-méthylène-4-prégnène-3,20-dione.

3. La 17α-hydroxy-1α,2α-méthylène-6-méthylène-4-prégnène-3,20-dione.

4. La 17α-acétoxy-6α-méthyl-1α,2α-méthylène-4-prégnène-3,20-dione, la 6α-méthyl-1α,2α-méthylène-17α-propionyloxy-4-prégnène-3,20-dione, la 17α-butyryloxy-6α-méthyl-1α,2α-méthylène-4-prégnène-3,20-dione, la 6α-méthyl-1α,2α-méthylène-17α-valéryloxy-4-prégnène-3,20-dione, la 17α-hexanoyloxy-6α-méthyl-1α,2α-méthylène-4-prégnène-3,20-dione, la 17α-méthoxyméthoxy-6α-méthyl-1α,2α-méthylène-4-prégnène-3,20-dione.

5. La 17α-hydroxy-6α-méthyl-1α,2α-méthylène-4-prégnène-3,20-dione.

6. Compositions pharmaceutiques, caractérisées par une teneur en composés suivant l'une des revendications 1 à 5.

7. Procédé de préparation de 1α,2α-méthylène-6-méthylène-prégnènes et de 1α,2α-méthylène-6α-méthyl-prégnènes répondant à la formule générale I

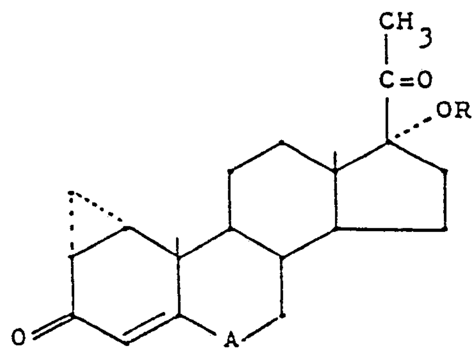

(I)

dans laquelle A représente un groupement

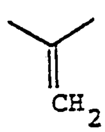 ou 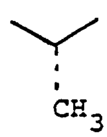

et R représente un atome d'hydrogène ou un groupe acyle ou alcoxy-méthyle, caractérisé en ce qu'on traite des 1α-chlorométhyl-6-méthylène-prégnènes de la formule générale II

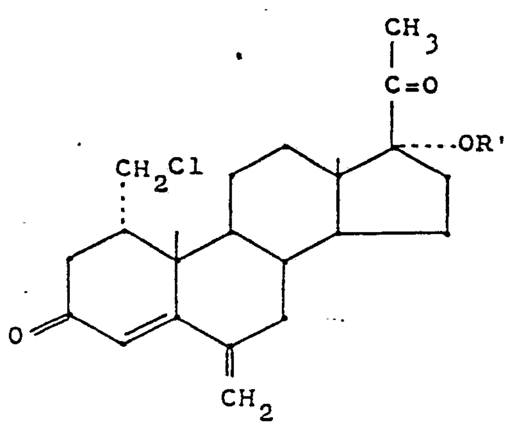

(II)

dans laquelle R' représente un groupe acyle ou alcoxyméthyle, avec une base, le noyau de 1,2-méthylène étant formé par élimination de HCl, et en ce qu'on convertit éventuellement ensuite le composé 6-méthylène en le composé 6α-méthyle par une réaction de transfert d'hydrogène catalytique et un traitement acide ou en ce qu'on effectue en une seule étape l'élimination de HCl pour former le composé 1,2-méthylène et l'hydrogénation pour former le composé 6α-méthyle dans les conditions de la réaction de transfert d'hydrogène catalytique avec traitement acide et en ce qu'on hydrolyse éventuellement ensuite un groupe 17α-acyloxy.

**Revendication** (pour l'Etat contractant AT)

Procédé de préparation de 1α,2α-méthylène-6-méthylène-prégnènes et de 1α,2α-méthylène-6α-méthyl-prégnènes répondant à la formule générale I

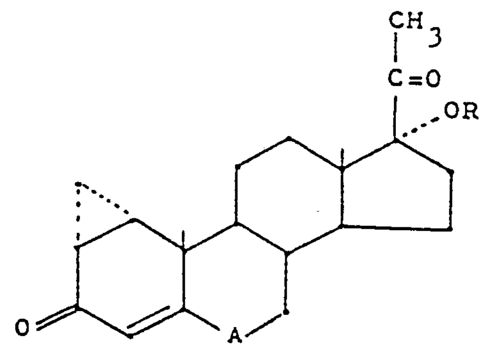

(I)

dans laquelle A représente un groupement

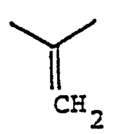

ou

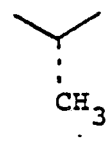

et R représente un atome d'hydrogène ou un groupe acyle ou alcoxyméthyle, caractérisé en ce qu'on traite des 1α-chlorométhyl-6-méthylène-prégnènes de la formule générale II

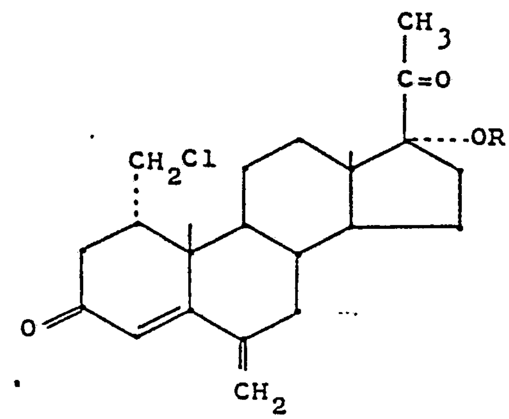

(II)

dans laquelle R' représente un groupe acyle ou alcoxyméthyle, avec une base, le noyau de 1,2-méthylène étant formé par élimination de HCl, et en ce qu'on convertit éventuellement ensuite le composé 6-méthylène en le composé 6-méthyle par une réaction de transfert d'hydrogène catalytique et un traitement acide ou en ce qu'on effectue en une seule étape l'élimination de HCl pour former le composé 1,2-méthylène et l'hydrogénation pour former le composé 6α-méthyle dans les conditions de la réaction de transfert d'hydrogène catalytique avec traitement acide et en ce qu'on hydrolyse éventuellement ensuite un groupe 17α-acyloxy.